# EUROPEAN PATENT APPLICATION

(11) **EP 0 884 165 A2**
(43) Date of publication of application: **16.12.1998**
(21) Application number: 98110422.7
(22) Date of filing: 08.06.1998
(51) Int. Cl.: B29D 7/01, C08J 5/18

(54) **Polyolefin film and its use for producing personal hygiene products**

(30) Priority: 09.06.1997 IT MI971351
(71) Applicant: Nuova Pansac S.p.A., 20124 Milano (IT)
(72) Inventor: Fontana, Luigi, 20131 Milano (IT); Bortoletto, Graziano, 31059 Zero Branco (TV) (IT)
(74) Representative: Savi, Alberto

(57) **Abstract**

The invention relates to an embossed polyolefin film, suitable for producing personal hygiene products, which presents an improved pleasantness to the touch.

## Description

The object of this invention is a polyolefin film and its use for producing diapers for babies and incontinents, ladies' sanitary towels, overalls, medical bandages, plasters, absrobant towels and other items of apparel designed for personal hygienic protection.

The use of extruded polyethylene or other polyolefin films are not in favour with the users due to the "plastic" feeling felt when handling the products and thus coming in contacts with the hands or skin in general.

In the case of diapers for incontinents, the friction between the polyethylene film and the fabric of trousers and/or rubber garments generates annoying noises and/or embarrassement to the wearer.

In order to overcome these problems, a known technique suggests applying an embossing pattern on the surface of the film, which usually takes the form of rhombic or hemispherical imprints, uniformly distributed in well defined manner.

However, the results obtained by such an embossing process have been rather limited.

Another known technique suggests coupling or laminating the polyethylene film with a layer of non-woven polypropylene fabric actually conveying a highly pleasant touch.

On the other hand, these films exhibit a number of shortcomings during their production, as well as high manufacturing costs.

Moreover, the non-woven fabrics inherently tend to absorb dust, thus acting as microbial receptors.

The main purpose of this invention is to develop a film designed for use as a support and/or water barrier for the production of diapers, ladies' sanitary towels and other personal hygiene products, marked by modest manufacturing cost, low microbial and dust retention, highly pleasant touch and negligible "noisiness" when coming in contact with clothing.

Such a film possesses the characteristics outlined in accordance with claim 1.

In its preferred embodiment, the film presents the characteristics of the dependent claim 2.

An embodiment of this invention will now be described, for purely exemplary purposes, with reference to the attached drawings, in which:

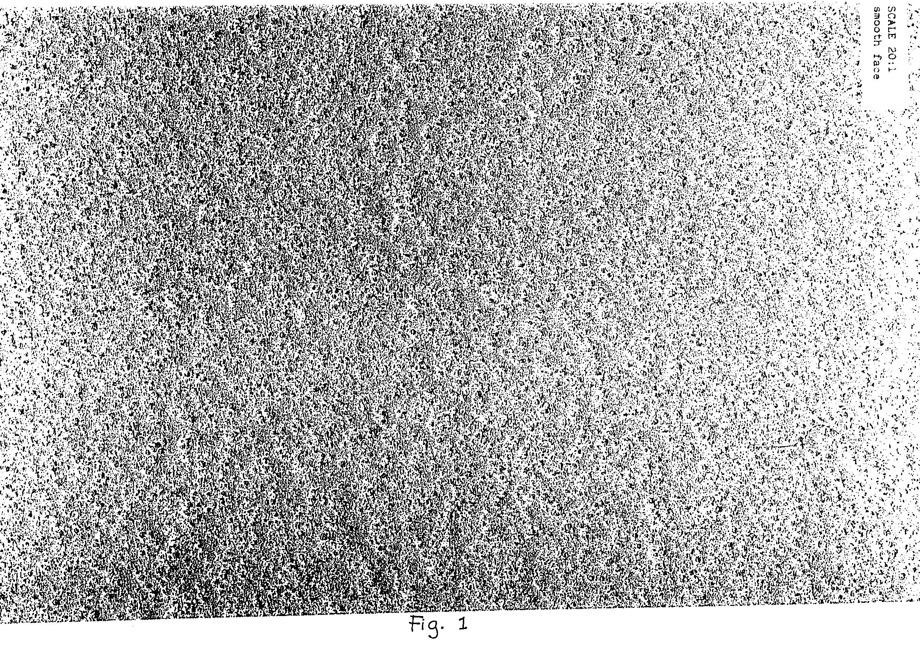

- Figure 1 is a front view, on a 20:1 scale, of the"smooth" face of the film;

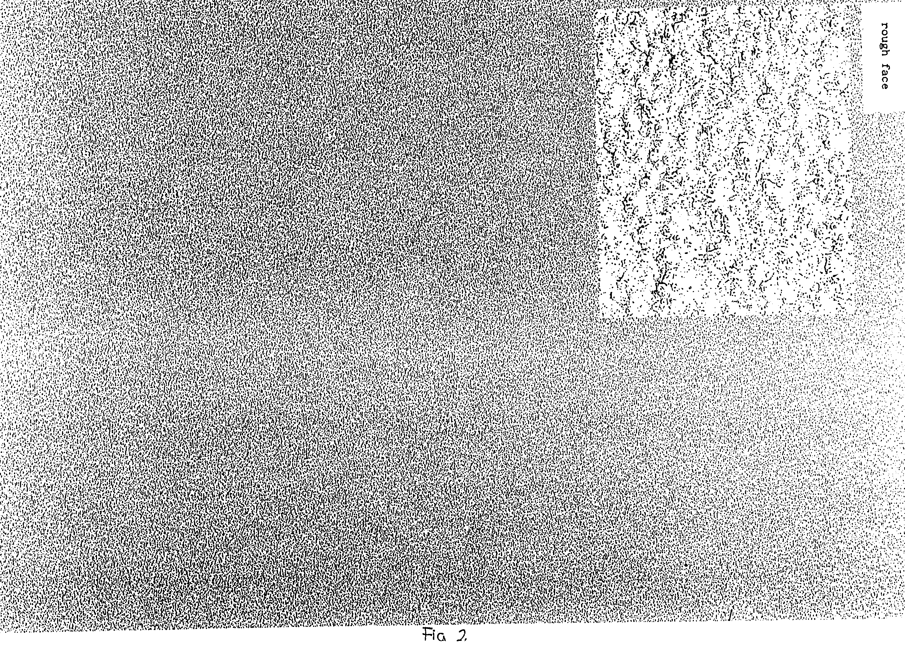

- Figure 2 is a front view, on a 1:1 scale but with a detail enlarged on a 20:1 scale, of the "rough" face of the film; and

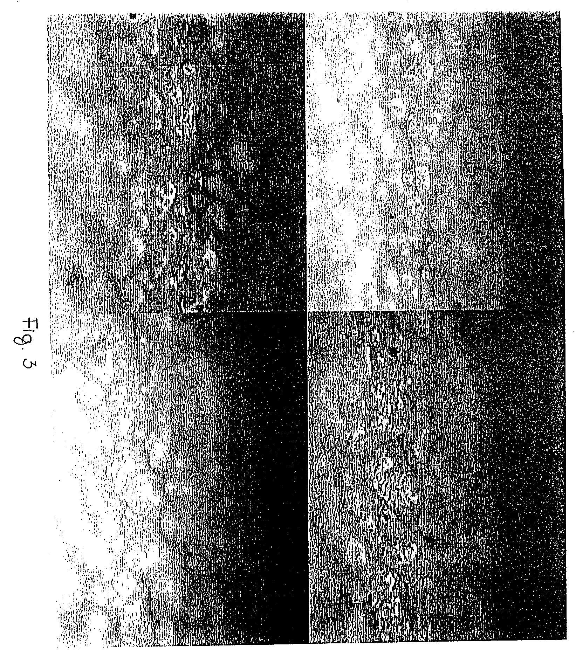

- Figure 3 represents four views, from an equal number of angles, on a 200:1 scale of the "smooth" face of the film.

The film of this invention is an extruded film formed essentially by polyolefins, for example polyethylene obtained by several systems known as flat-head processes (according to the so-called "cast" method) or bubble processes (according to the so-called "blown" method).

The feature of the film is that it presents a "rhinocerus hide-like" embossing pattern.

The term "rhinocerus hide-like" in this invention means a type of embossing in which the patterns (crests and grooves) exhibit irregular shapes and dimensions and are distributed over the surface in a casual manner, without a precise design.

In detail the polyethylene film is introduced between two rollers in a plastic state; a smooth roller made of rubber or other resilient material and a second one made of steel coated with a particular, mechanically resistant material bearing the above embossing pattern, to as to provide the finished product with a tactile feeling similar that of cloth.

The steel roll used in the mentioned embossing process has a roughness Ra, in every direction, in the range of 0.5 and 30. The amplitude of the irregularities (crests and grooves) varies from a maximum of 150 micron to a minimum of 20 micron, the form and distribution of the imprints is irregular (see the figures attached).

The frequency of these imprints varies from about 20 to 150 units per linear cm.

The embossing depth on the film can be adjusted by modifying the chemical composition of the film and/or by adjusting the pressure of the embossing rollers.

The embossing ratio, meaning the ratio between the apparent and the true thickness of the fim may vary from 1.5 to 8.

In one possible embodiment, the smooth film presents an average thickness of 25 µm, and an average apparent thickness of about 100 µm after the embossing process.

It has been noted that the embossing process described above allows drastically reducing the "plastic" feeling on a touch.

In addition, the film obtained in this manner has a lower "noisiness" than that of the films produced by traditional embossing systems.

The film described in this invention preferably exhibits the following chemical composition:
- from about 5% to about 65% by weight of a linear, medium density polyethylene (LLDPE);
- from about 5% to about 95% by weight of a linear, low density polyethylene (LDPE); and
- from about 1% to about 50% by weight of a linear,very low density polyethylene (VLDPE).

The term linear, medium density polyethylene in this invention means a polyethylene with a density above 0.925 and equal or lower than 0.939 kg/dm³.

The term linear, low density polyethylene in this invention means a polyethylene with a density above 0.915 and equal or lower than 0.925 kg/dm³.

The term linear, very low density polyethylene in this invention means a polyethylene with a density equal or lower than 0.915 kg/dm³.

The chemical composition described above allows to advantageously enhance the feeling to the touch of the film.

The particularly outstanding result provided by the new formulation of the film and by the embossing can be summarized in the fact that the film surface offers a friction coefficient in the range of 0.4-1.5 (ASTM standard D-188A-78), which attests the achievement of an an improved pleasantness to the touch.

The film can be made white (at various degrees of coverage) by using titanium dioxide and calcium carbonate or colored by using color additives and pigments in suitable quantities, according to a known technique.

The film thus produced has proved to be satisfactory as a support and/or an aqueous barrier in manufacturing countless products, such as for example:
Diapers for babies and incontinents, ladies' sanitary napkins, medical bandages, plasters, absorbant towels, canvases, disposable rags, tablecloths, and overalls or bodysuits for medical purposes.

The skilled person may of course envision further uses of the film described on this occasion, but all of them falling within the scope of protection of this patent.

## Claims

1. A polyolefin film, in particular for producing personal hygiene products, characterized by the fact that it offers a"rhinocerus hide-like" embossing pattern.

2. A film according to claim 1, having the following chemical composition:
- from about 5% to about 65% by weight of a linear, medium density polyethylene (LLDPE);
- from about 5% to about 95% by weight of a linear, low density polyethylene (LDPE); and
- from about 1% to about 50% by weight of a linear,very low density polyethylene (VLDPE).

3. A use of a polyolefinic film according to the claims 1 or 2, to be used as a support and/or water barrier for personal hygiene products.

4. A use of a polyolefinic film according to the claims 1 or 2, for the production of diapers for babies and incontinents and ladies' absorbants.

5. A use of a polyolefinic film according to the claims 1 or 2, for the production of apparel, in particular for hospital uses.

6. A use of a polyolefinic film according to the claims 1 or 2, to be used as a support for the production of disposable absorbant towels.
